# EUROPEAN PATENT SPECIFICATION

(11) **EP 1 909 816 B1**
(45) Date of publication and mention of the grant of the patent: **15.09.2010**
(21) Application number: 06796248.0
(22) Date of filing: 03.08.2006
(51) Int. Cl.: A61K 38/16, A61K 35/74, A61K 31/485, A61P 25/00, A61P 25/04, A61P 25/06, A61P 25/28

(54) **CNF1 BACTERIAL TOXIN PROTEIN FROM E. COLI AS ANALGESIC**
CNF1 BACTERIELLES TOXIN-PROTEIN AUS E. COLI ALS ANALGETIKUM
CNF1 TOXINE PROTÉIQUE BACTÉRIELLE DE E. COLI EN TANT QU'ANALGESIQUE

(30) Priority: 05.08.2005 IT RM20050422
(43) Date of publication of application: 16.04.2008
(73) Proprietor: CONSIGLIO NAZIONALE DELLE RICERCHE, 00185 Roma (IT); ISTITUTO SUPERIORE DI SANITA', 00161 Roma (IT)
(72) Inventor: MALORNI, Walter, I-00161 Roma (RM) (IT); FIORENTINI, Carla, I-00161 Roma (RM) (IT); FABBRI, Alessia, I-00161 Roma (RM) (IT); FALZANO, Loredana, I-00161 Roma (RM) (IT); STRAFACE, Elisabetta, I-00161 Roma (RM) (IT); PAVONE, Flaminia, I-00185 Roma (RM) (IT); LUVISETTO, Siro, I-00185 Roma (RM) (IT); MARINELLI, Sara, I-00185 Roma (RM) (IT)
(74) Representative: Gitto, Serena
(86) International application number: PCT/IT2006/000596
(87) International publication number: WO 2007/017914

(56) References cited:
- EP-A- 1 580 195
- EP-A2- 1 570 856
- WO-A-2006/105998
- WO-A2-01/25397
- US-A1- 2002 037 833
- FALZANO LOREDANA ET AL: "Induction of phagocytic behaviour in human epithelial cells by Escherichia coli cytotoxic necrotizing factor type 1" MOLECULAR MICROBIOLOGY, vol. 9, no. 6, 1993, pages 1247-1254, XP002416809 ISSN: 0950-382X

## Description

The present invention concerns new uses in medical field of the dermonecrotizing protein factor of bacterial origin being protein CNF1 toxin of Escherichia coli for the therapy of the pain.

The better knowledge about the basic mechanisms of the nociceptive transmission and clinical experience resulted in a characterization of the various types of pain and research of more suitable and specific treatments according to the various algia syndromes.

The present analgesics for the pain therapy in the various pathological conditions (inflammatory acute or chronic, post-operative pain, neurodegenerative pathologies, neoplasias) can be classified in two main categories, i.e. non steroid anti-inflammatory drugs (FANS), and analgesic narcotics. FANS (for example, aspirin) inhibit the prostaglandin synthesis and reduce the sensitivity of nerve endlings. While FANS are mainly used for the treatment of the inflammatory and mild pain, the analgesic narcotics are used also for the therapy of the severe pain. The latter ones act at level of opioid µ (mu) receptors in the central and peripheral nervous system. This large naturally occurring drug class usually used for the pain treatment include, among the others, opium derivatives, morphine, heroin, methadone. There are also synthetic drugs resulting in the same pharmacological effect as opium more usually used as for example the meperidine, fentanyl, surfentanyl, alfentanil, and remifentanil.

Both the analgesic categories currently used in anti-pain and anti-inflammatory therapy are associated to the occurrence of various side-effects. As an example, FANS are well known due to their renal, hepatic and mainly gastrointestinal toxicity (gastric and intestinal ulcers). Another problem results from FANS pharmacodynamic and pharmacokinetic properties whereby at a certain threshold of therapeutic activity also increasing the FANS doses it is not possible to increase further the pain-relieving effect.

But, above all, the opioid use, although preferred drugs for the treatment of the pain in advanced tumor suffering or post-operative course patients, is associated with serious side-effects. In fact, through their activity mechanism involving the formation of a bond to µ receptors, opioids induce positive therapeutic effects like analgesia, together with undesired effects, as for example, the physical and psychological dependency, hypoxia, psychoactive effects, respiratory depression, conscience loss and death too. Due to the elevated risk of hypoxia and respiratory depression the physicians prefer not to use the appropriate doses of opioids for the treatment of patients suffering from acute or chronic pain.

Moreover their dosage must continuously be increased in order to maintain their analgesic effect always at a given level, as result of complex pharmacokinetics thereof. Furthermore, the pain is highly variable and subjective syndrome and therefore the opioid response varies from patient to patient. As result, different doses of analgesics for the treatment of the individual patients are necessary. Therefore it is highly desirable to allow the recipient patients vary the analgesic amount, guaranteeing an extended duration of the effect, without the occurrence of serious side effects.

Moreover, some algia syndromes as neuropathies or nervous lesions are not sensitive to the treatment both with the opiate and FANS, whereby the development of a therapeutic approach for this type of pain is very interesting (Woolf C. et al., 1999).

In the light of above it is apparent the need of providing new specific and effective active principles for the therapeutic treatment of the pain, particularly acute or chronic, neoplastic inflammatory or post-operative condition pathologies.

The authors of the present invention have surprisingly found that the dermonecrotizing protein factor being CNF1 bacterial protein toxin (cytotoxic necrotizing factor of type 1) produced from different *Escherichia coli* strains has a pronounced analgesic activity.

Therefore their use in medical field like anti-pain, particularly for pathological inflammatory acute and/or chronic and post-operative conditions, has been suggested. Recently therapeutic uses as analgesics against various natural toxins like polypeptide HWAP-I from *Selenocosmia huwena* spider, as described in EP 1161951 or against *Clostridium* botulinum toxin for burn pain, described in patent application EP 1550456 have been shown.

CFN1 toxin from *Escherichia coli* (110 kD protein) selectively and permanently activates Rho family regulatory G proteins mainly involved in the regulation of cytoskeletal actin and expression of membrane receptors. Such proteins are involved in the mechanisms of neuropathic pain whereby the use of CNF1 toxin is useful for the treatment of such a pain not very sensitive to FANS and opioids. The neuropathic pain is in fact very diffused and can occur following dental or ophthalmic surgery, burns, diabetic neuropathy, neuralgia, arthritis and like.

Moreover, based on some immunofluorescence data wherein the modulation by CNF1 of opioid µ receptors has been observed, the authors have found that CNF1 toxin can be advantageously employed in combination to an opioid like morphine, thus allowing the reduction of the opioid doses to be administered and reducing the side effects.

Therefore, the present invention concerns the use of CNF1 bacterial protein toxin from a native or recombinant *Escherichia coli* strain, active portion or variant thereof for the preparation of an analgesic medicament.

Preferably said *Escherichia coli* strain is chosen from the group consisting of intestinal and extra intestinal toxigenic pathogens, preferably it is an uropathogen, still more preferably uropathogen J96 (O4: K6) strain.

According to a preferred embodiment said active portion is the active site of CNF1 bacterial protein toxin from 720 to 1007 amino acids (Lemichez et al., 1997) of CAA50007 number CNF1 reference sequence (Gene Bank) (Falbo et al., 1993).

A further object of the present invention is the use of a nucleic acid construct comprising an oligonucleotide sequence encoding for CNF1 bacterial protein toxin or active portion thereof, as above defined, for the preparation of analgesic medicament.

The invention preferably refers to both the use of nucleic acid construct as above defined or above described CNF1 bacterial protein toxin, for the preparation of an analgesic drug for the treatment of acute or chronic, inflammatory neoplastic pathologies, or post-operative conditions.

According to a particularly preferred embodiment of the present invention said inflammatory pathology is neuropathy.

According to a further preferred embodiment of the invention said inflammatory chronic pathology is neurodegenerative pathology.

The use of above defined construct or CNF1 bacterial protein toxin constitutes a further object of the present invention, in combination with at least an analgesic. According to a preferred embodiment of the present said analgesic is an opioid from the group consisting of morphine, codeine, dihydroxycodeine, diacetylmorphine, hydrocodone, hydromorphone, levorphanol, oxymorphone, alfentanyl, buprenorphine, butorphanol, fentanyl, sufentanyl, meperidine, methadone, nabulphine, propoxyphene, pentazocine; or pharmacologically acceptable salts thereof; preferably it is morphine. This use in combination would allow to reduce the opioid dose reducing the possible side effects. The use in combination means both coadministration within the same formulation and two different formulations sequentially administered via the same way or not.

Therefore another object of the present invention is the pharmacological combination of active principles comprising the CNF1 bacterial protein toxin or nucleic acid construct as above defined and at least an analgesic, preferably an opioid selected from the previously defined group, still preferably morphine.

According to an alternative embodiment the pharmaceutical composition according to the invention comprises the pharmaceutical combination as above defined, together with one or more pharmaceutically acceptable adjuvants and/or excipients. Preferably in the above defined composition according to the invention the toxin amount is variable in the range from 10 to 100 ng/ml/kg of patient body weight.

The administration of the active protein or portion(s) thereof can be carried out by different mode including intrathecal or subcutaneous.

The present invention refers to the use of above defined pharmacological combination or pharmaceutical composition for the treatment of acute or chronic, inflammatory neoplastic pathologies, or post-operative conditions. According to a particularly preferred embodiment of the present invention said inflammatory pathology is neuropathy. According to a further preferred embodiment of the invention said inflammatory chronic pathology is neurodegenerative pathology.

A process for the preparation of above defined CNF1 bacterial protein toxin may comprise the following steps:
a) culture of *Escherichia coli* strain, preferably pISS 392 strain, centrifugation;
b) preparation of bacterial lysate and precipitation of supernatant in buffer solution;
c) buffer dialysis
d) purification by means of chromatography, preferably ion exchange and/or gel filtration chromatography.

The medium suitable to be used for culture of step a *E. coli* is known in the art, and it is preferably LB medium. Step b) cell lysis preferably is carried out by sonication and the precipitation of the precipitated supernatant occurs in ammonium sulphate. Finally, after the purification, CNF1 is frozen in small aliquots.

The peptide **characterized in that** it is the active site (triade consisting of Cys 866 and His 881 as described by Buetow et al., 2001) of CNF1 bacterial protein toxin has the following amino acid sequence:
from 720 to 1007 amino acid of CAA5007 reference sequence (Gene Bank) (Falbo et al., 1993).

It is an object of the present invention the use of antibodies specific for CNF1 bacterial protein toxin as defined above for the preparation of an antidote to inhibit the peptide activity on proteins G where an analgesic temporary effect is desired.

These antibodies can be monoclonal or polyclonal; alternatively also antibody fragments, like Fab fragments can be used.

The present invention now will be described by illustrative but not limitative way according to preferred embodiment thereof with particular reference to the enclosed drawings, wherein:

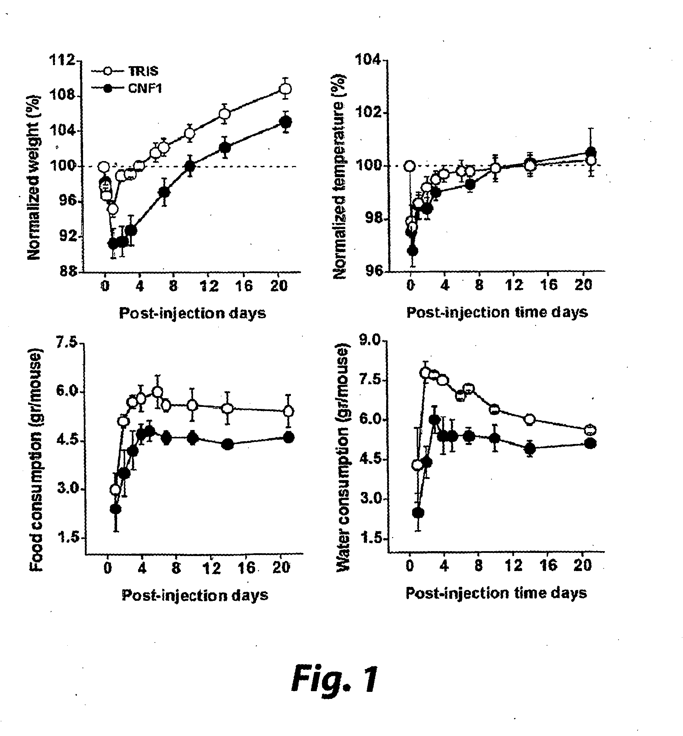

figure 1, shows the plots versus time of the body weight, temperature, water and food consumption after TRIS and CNF1 (1: 1000) intracerebroventricular injection in CD1 male rats; the body weight is reported as normalized weight with respect to body weight before the intracerebroventricular injection;

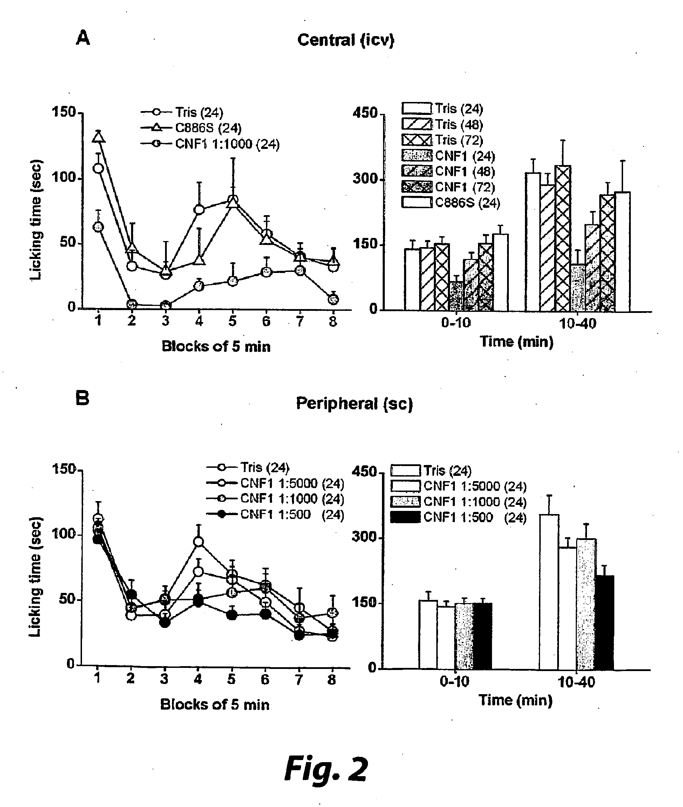

figure 2 shows the plot versus time of the licking response (left side) and licking cumulative time (right side) during step 1 (0-10 minutes) and step 2 (10-40 minutes) for the formalin test; panel A shows licking response to TRIS and CNF1 (1: 1000), administered 24, 48 or 72 hours before the formalin test; C886S, inactive mutant form of CNF1, has been injected 24 hours before the test; panel B shows the peripheral effects on the licking response to TRIS and CNF1 injected 24 hours before the formalin test;

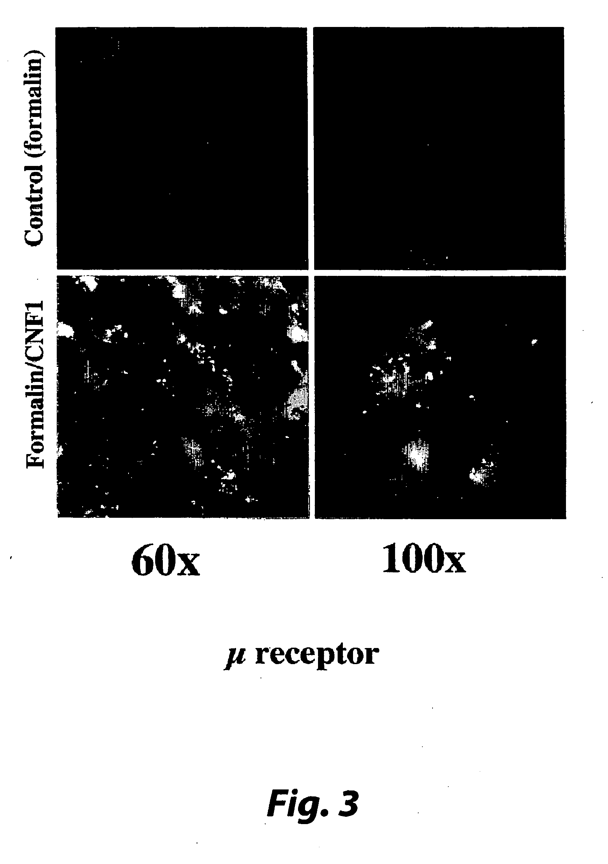

figure 3 shows the positive regulation of the opioid receptor in CNF1 treated samples;

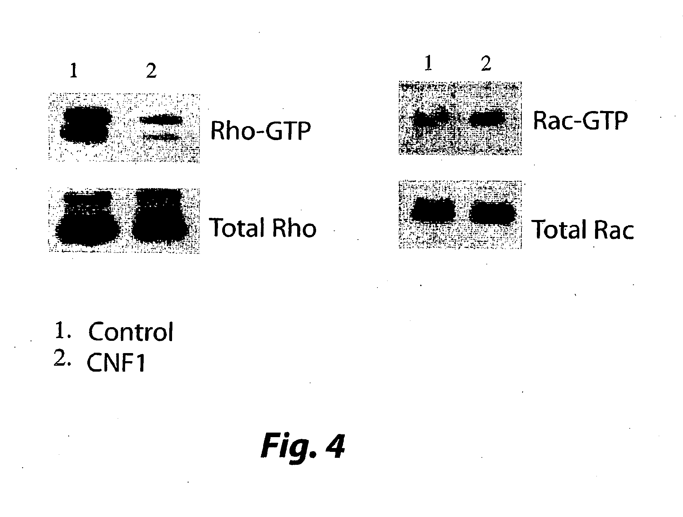

figure 4 shows Western blot of the Rac activation in different samples;

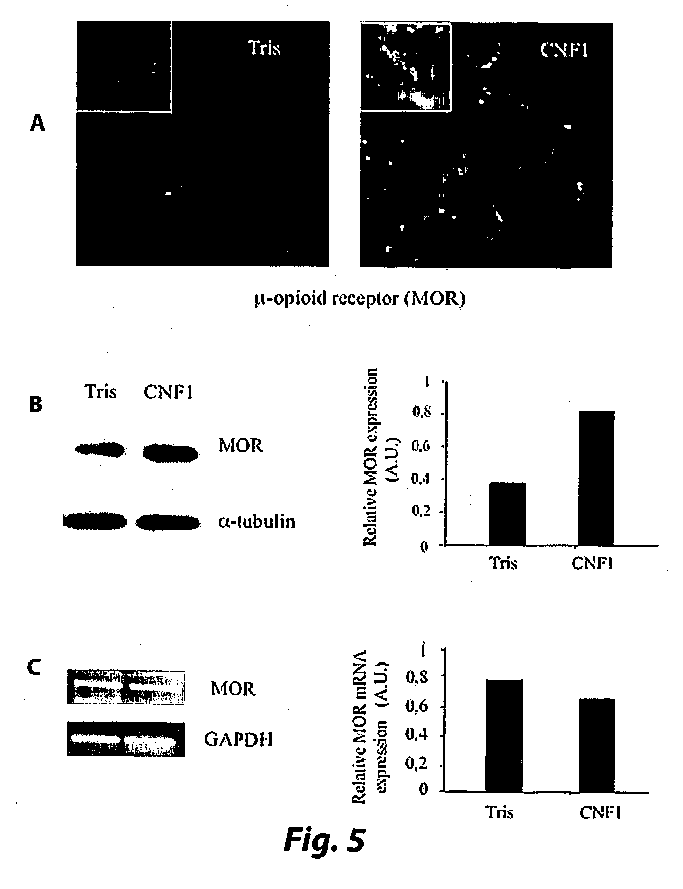

figure 5 shows the up-regulation of the opioid µ receptors resulting from CNF1; panel A shows fluorescence microscopy and confocal (insert) analyses for sections of PAG area for control (left) or CNF1 treated (right) rats, respectively; the sections are labelled for the opioid µ receptor; panel B shows Western blot analysis of opioid µ receptor of brain lysate from PAG area; panel C shows RT-PCR analysis of the mRNA from brains (area PAG) of control or CNF1 treated rats, respectively; The amount of mRNA for opioid µ receptor is the same for control and treated rats (representative densitometric analysis on the right);

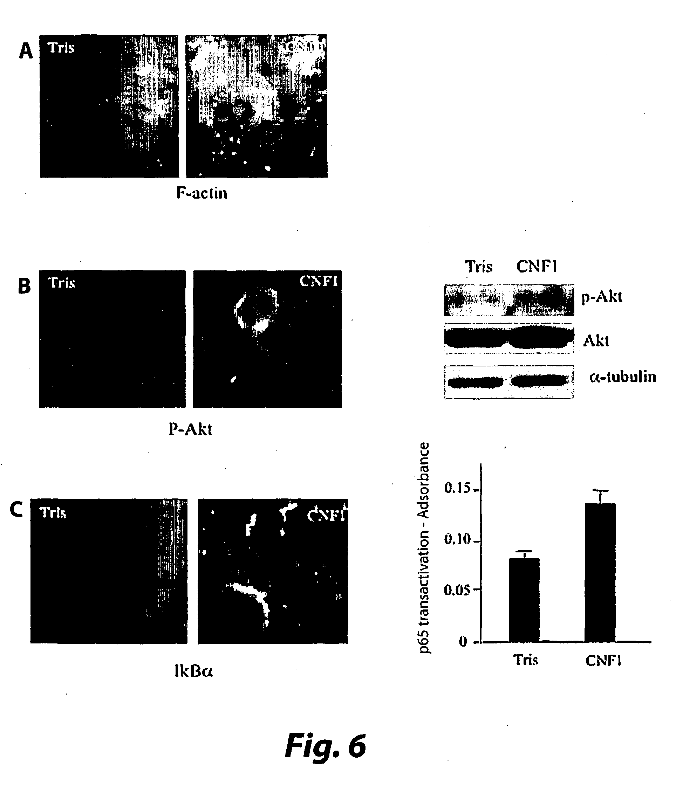

figure 6 shows CNF1 activated intracellular pathway; panel A shows fluorescence microscopy analysis for sections of PAG area for control (Tris) or CNF1 treated (right) rats, marked to point out F-actin; CNF1 injection results in an increase of F-actin expression and "patches" occurrence in the cellular periphery; magnification 3000 x; panel B shows *Akt* activation and confocal microscopy and Western blot analyses of p-Akt in PAG area sections from Tris or CNF1 treated rat brains: the redistribution, analyzed by fluorescence microscopy (left panel), and the expression increase, analyzed by Western blot (right panel), of p-Akt in PAG area of CNF1 treated rats; α-tubulin labelling confirms the loading of equal protein amount; panel C shqws the *NF-kB* activation through the confocal microscopic analysis of IkBa in PAG area of Tris or CNF1 treated rat brain sections: the exposure to the CNF1 results in an apparent redistribution of IkBa in the cellular cytoplasm. (left panel); the graph shows also CNF1 injection induced NF-kB-p65 trans-activation (right panel).

### EXAMPLE 1: Identification of the analgesic activity of CNF1 bacterial toxin in animal models

The authors have demonstrated in test animals that the CNF1 induced Rac activation plays an effective and key role for the modulation of inflammatory pain. The mechanisms underlying this analgesic effect are probably associated to the cytoskeleton dependent synaptic traffic and/or neurotransmitter release.

One of most used techniques to induce the extended pain is the formalin test, wherein the licking response induced in the animals after the injection of a formalin solution in the rear leg is considered a pain index. The nociceptive response, considered as the consequence of the stimulation of the nociceptors and inflammatory processes shows two distinct, i.e. early and late, steps differing in the mechanism of action and the drug sensitivity. Although various pharmacological studies have extensively investigated about the mechanisms and ways of signal transmission involved in the inflammatory pain, the intracellular mechanisms associated to the onset of the inflammatory pain are still unknown. Particularly, subcellular structures involved at the beginning of the pain have been studied.

In the present study the authors have investigated the effects of CNF1 bacterial toxin on inflammatory pain in rats using the formalin test. CNF1 is a protein toxin suitable to activate in a powerful, specific and permanent way Rac-1 molecule. The latter belongs to small GTPase Rho protein family comprising three main subfamilies, i.e. Rho, Rac and cdc42. It has been involved in the dynamic modifications of the microfilament system. Particularly, the movement of actin filaments is controlled by these regulatory protein class which in turn direct the formation of stress fibers (Rho), pseudopods (Rac) or phyilopods (cdc42). The authors demonstrated that the subcellular activity of this toxin through Rac supported activation can modulate the pain response.

### MATERIALS AND METHODS

### Formalin Test

Different systems for the study of CD1 rat male behavior (Charles River, Como) together with various biochemical, like pull down and western blot, and immunohistochemical techniques in cerebral tissue samples.

In order to carry out the intracerebroventricular injection (icv), rats have been implanted with intracranial cannulas under anaesthesia using a mixture of ketamine (100 mg/Kg) and xilazine (5 mg/Kg, intraperitoneally), as previously described (Luvisetto et al., 2004; Luvisetto et al., 2003). The rat head has been fixed in a stereotaxic apparatus and an incision has been carried out in the atlanto-occipital membrane. Stainless steel guide cannulas have been implanted according to stereotaxic coordinates (anteroposterior, AP 0,0, mediolateral, ML ± 1,0 mm, to bregma) of the murine brain Atlas (Franklin and Paxinos, 1997). Stainless steel injection cannulas, connected by means of polyethylene tubes to an infusion pump, have been inserted in lateral cerebral ventricles (dorsoventral, 2,5 DV mm to bregma).

CNF1 has been administered in a volume of 1 µl/rat (dose of 1 µl/min as single injection). After icv injection the rat body weight, water and food consumption have been measured every day. The locomotion activity has been also tested in order to exclude a significant motorial alteration for CNF1 injected rats.

The formalin test has been carried out 24, 48, and 72 hours after CNF1 or TRIS (control groups) icv injection in different groups of rats. The day of the test, one animal at a time has been placed for an hour in a plexiglass standard cage (30x12x13 cm) used as observation room. After this adaptation period, 20 µl of formalin solution (5% salt solution) have been injected subcutaneously on the dorsal surface of the rat posterior leg using 36 bore needle microsyringe. The rats have been placed again in the room and the observation period started. The licking activity, i.e the total amount of time during which the animal licks or bites the injected leg, has been considered as pain index. The licking activity has been recorded in continuous for 40 minutes and calculated in blocks of 5 consecutive minute periods. A mirror localized behind the cage allowed a undisturbed observation of the rat.

Other groups of rats have been subjected to subcutaneous administration (sc) of CNF1 (1: 500, 1:1000, 1:5000) or Tris on the surface of right posterior leg in a volume of 10 µl/rat using 26 bore needle microsyringe.

The activation state of Rho GTPase has been tested by pull down experiments. Tissue homogenates have been prepared using the following extraction buffer: 50 mM Tris pH 7,4, 1 mM EDTA, pH 8, 0.5% NP₄0, 150 mM NaCl, 10% glycerol, 1 µg/ml of aprotinin and leupeptin, 250 µg/ml PMSF. Homogenate has been purified by centrifugation at 14000 rpm at 4°C for 5 minutes. Pull down test has been carried out using 0,5 mg of homogenate and 60 mg of GST-PAK70-106. Proteins have been separated on 12% SDS PAGE and then transferred on polyvinylidiene difluoride membranes (BIO-RAD). The Rac - GST-PAK70-106 bond and total Rac have been determined by Western blot analysis as previously described (Boyer et al., 2003).

Immunohistochimical analysis has been carried out using 10 µm thick cryosections labelled with monoclonal antibodies against opioid receptors and actin filaments.

Particularly, as for opioid receptor labelling after re-hydration in PBS buffer, sections have been incubated for 30 minutes at 37°C with anti-mu (Chemicon, 1:20). After several PBS washings, samples have been incubated with anti-rabbit FITC (Sigma, 1:20) for 30 minutes at 37°C.

In order to label actin filaments, the re-hydratated sections have been incubated for 30 minutes at 37°C with phalloidin-FITC (Sigma, 1:300).

After washings with PBS buffer, all the samples are mounted with PBS/glycerol and observed using a fluorescence microscope.

### RESULTS

All the standard tested behavioral parameters, as, for example, body weight, temperature, water and food consumption, and the locomotorial activity (not shown), rapidly diminished immediately after icv injection independently from the type of treatment, as shown in Figure 1. While the rats recover the relative values of the body temperature during few days and differences between the control and CNF1 treated groups are not observed, the recovery of the body weight and food and water intake is much slower and the CNF1 treated rat body weight in any case remains lower than Tris treated rats.

Powerful analgesic activity against inflammatory pain after CNF1 icv injection is observed (Figure 2, panel B), as indicated by the meaningful reduction of the licking response to formalin test. This anti-inflammatory activity is more apparent in rat injected with CNF1 24 hours before the test. Due to this reason such a time interval has been chosen for the experiments wherein CNF1 toxin is administered by subcutaneous peripheral way. It is interesting to point out that both the early and late steps, corresponding to the phasic and tonic pain, are conditioned by CNF1 when injected 24 hours before the test.

The anti-nociceptive effect has been also observed after the peripheral administration (Figure 2, panel A), but in this case only the late step of the test has been meaningfully reduced by the CNF1 treatment.

As for biochemical and immunohistochemical experiments, Rac mediated remodelling of citoskeleton and actin filaments, negative regulation of µ receptors (pain opioid receptors) (Figure 3) and GTPase Rac activation (Figure 4) have been also observed.

### ESEMPIO 2: Studies on the action mechanism and molecular pathway involved in CNF1 analgesic activity

Among the pain receptors, the opioid µ receptor family is one of the most important. The authors have verified whether the distribution and expression of such receptors could be modified in a brain area particularly involved in the nociception, i.e. periacqueduttal area (PAG).

### MATERIALS AND METHODS

### Fluorescence microscopy

Control and CNF1 intracerebroventricular administered animals, have been sacrificed 24 hours later, their brains removed and quickly frozen at - 80°C. 10 mm thick sections of PAG area have been obtained at the cryostate and after fixation in PBS 4 % paraformaldehyde the same have been incubated (30 min at 37°C) with different antibodies:
i) µ anti-receptor polyclonal antibody (Abcam Cambridge, UK);
II) anti p-AKT polyclonal antibody (Santa Cruz Biotechnology, CA, USA);
III) anti IkBa monoclonal antibody (Sigma).

After PBS washings, the sections have been incubated with the appropriated FITC OR TRITC conjugated secondary antibody for 30 min at 37°C.

For F-actin and nucleus labelling, the sections have been incubated for 30 min at 37°C with FITC-phalloidin or Hoechst 33258 (Sigma, St.Louis, MO), respectively. Samples have been analyzed using Olympus BX51 fluorescence microscope equipped with "Charge-coupled device CCD camera" (Carl Zeiss). The same sections have been examined using Leica TCS 4D confocal microscope.

### Assay on NF-kBp65 activation

In order to study the NF-kB activation "NF-kBp65/NF-kBp50 transcription factor assay kit" (TransAM, Active Motif, California, USA) has been used according to the supplier instructions. Moreover localization of NF-kBp65 complex has been analyzed using fluorescence microscopy as above described.

### Western blot

PAG area brain portions have been lysated in "sample buffer" (50 mM Tris-HCl pH 6,8, 2% SDS, 10% glycerol) and maintained at 100°C for 5 minutes. After centrifugation at 10.000 rpm for 10 min the sample protein concentration and 20 µg of total protein have been separated on polyacrylamide gel and transferred on PVDF membranes. The membranes have been labelled midnight at 4°C with the following TBS-T+2% skimmed milk diluted primary antibodies
i) anti Rac monoclonal (Transduction Laboratories);
II) anti α-tubulin monoclonal (Sigma);
III) anti p-Akt (Santa Cruz Biotechnology, CA, USA);
iv) anti Akt (Santa Cruz Biotechnology, CA, USA);
v) anti µ receptor (Abcam Cambridge, UK).

After several washings, membranes have been incubated for 1 hour at room temperature with the appropriated HRP conjugated secondary antibody and the protein expression has been detected by ECL reaction.

### RESULTS

Fluorescence and confocal microscopy observations have evidenced a redistribution over the cellular surface of opioid µ receptors (Figure 5A, insert: confocal microscopy). Moreover western blot analysis indicated a twofold increase of these receptor expression (Figure 5B, left panel; densitomentric analysis in right panel). On the contrary, the amount of opioid µ receptor messenger remains unaltered, as by RT-PCR analysis (Figure 5C, left panel; densitometric analysis in right panel). These results suggest that the CNF1 regulatory activity on these receptors is carried out at post-transcriptional level.

Since CNF1 causes Rac activation also in rat brain, the authors have verified whether within the downstream effects could be also a remodelling of the actin network, which is known to be also Rac controlled. Therefore the actin organization in sections from rat brain PAG area has been analyzed by fluorescence microscopy. Results showed that actin was re-organized in the brains from intracerebroventricularly CNF1 treated and not in Tris injected (Figura 6A, left) or heat inactivated or C866S CNF1 (data not shown) treated rats These results are consistent with the studies indicating the citoskeleton as potential regulator of the modifications associated with algia phenomena. In addition to the control of the actin citoskeleton GTPase Rac activation regulates the activation of a given number of kinases including Akt, a serine/threonine kinase whose activation requires the translocation to the plasmatic membrane. Akt activation, that is phosphorylation thereof (p-Akt), in turn results in the activation of NF-kB transcription factor involved in the regulation of various genes. Since it has been demonstrated that CNF1 activates NF-kB in isolated cells, we decided to analyze the p-Akt and NF-kB expression in brain samples. Ours results shown that p-Akt is present in cell citosol from Tris injected rats but translocates into the inner side of the plasmatic membrane after 24 hours from CNF1 injection (Figure 6B, left). In a similar way, IkBα, NF-kB inhibiting subunit, localizes in the plasmatic membrane of CNF1 icv injected rat brain (Figure 6C, left). It is known that the IkBα translocation into the membrane, together with phosphorylation thereof, allows the nuclear translocation of p65 NF-kB. Biochemical analyses indicated that the CNF1 is suitable to induce the Akt phosphorylation (Figure 6B, right) and p65 NF-kB transactivation (Figure 6C, right). Meffert and Baltimore recently have described that NF-kB family members are activated in the CNS and are associated with brain physiological functions. Therefore, the activation of the Akt/NF-kB pathway, that we have evidenced in PAG after CNF1 icv administration, could play a role in the observed analgesic effect and this is in agreement also with the NF-kB involvement in the pain mechanisms.

As a whole these results indicate CNF1 as an useful agent in the studies aimed to understand the subcellular mechanism on which the pain is based. Moreover they open new perspective for the pharmacological control of the inflammatory pain based on the modulation of pain perception by activity at level of intracellular pathways.

### BIBLIOGRAPHY

- Borghi V, Przwlocka B, Labuz D, Maj M, Ilona O, Pavone F. Brain Res. 2003 Aug 15; 981 (1-2):217.
- Buetow L., Flatau G., Labuz D., Maj M., Ilona O., Pavone F. Brain Res. 2003; 110(11):1205-13.
- Falbo V., Pace T., Picci L., Pizzi E., Caprioli A., Infect. Immun. 1993; 61(11):4909-14.
- Fiorentini C, Falzano L, Fabbri A, Stringaro A, Logozzi M, Travagliane S, Contamin S, Arancia G, Malorni W, Fais S. Mol. Biol. Cell. 2001; 12(7):2061-73.
- Fiorentini C, Matarrese P, Straface E, Falzano L, Donelli G, Boquet P, Malori W. Cell Death Differ. 1998; 5(11):921-9.
- Fiorentini C, Matarrese P, Straface E, Falzano L, Fabbri A, Donelli G, Cossarizza A, Boquet P, Malori W. Exp Cell Res. 1998; 242(1):341-50.
- Lemichez E, Flatau G, Bruzzone M, Boquet P, Gauthier M. Mol Microbiol. 1997; 24(5):1061-70.
- Luvisetto S, Rossetto O, Montecucco C, Pavone F. Toxicon 2003; 41:475-481
- Luvisetto S, Marinelli S, Rossetto O, Montecucco C, Pavone F. Behav Pharmacol (2004); 15:233-240.
- Malorni W, Quaranta MG, Straface E, Falzano L, Fabbri A, Viora M, Fiorentini C. J. Immunol. 2003; 171(8):4195-202.
- Nalepa I, Vetulani J, Borghi V, Kowalska M, Przwlocka B, Pavone F. J. Neural Transm. 2005 Feb. 22.
- Vetulani J, Pavone F, Przwlocka B, Borghi V, Nalepa I. J. Neural Transm. 2003 Nov; 110 (11): 1205-13.
- Woolf C. et al., Lancet 1999; 353: 1959-64).
- EP 1161951.
- EP 1550456.

## Claims

1. Use of CNF1 bacterial protein toxin from native or recombinant *Escherichia coli* strain, active portion or variant thereof, for the preparation of analgesic medicament.

2. Use according to claim 1, wherein said *Escherichia coli* strain is selected from the group of intestinal and extra intestinal toxigenic pathogens.

3. Use according to claim 2, wherein said strain is an uropathogen, preferably uropathogen J96 (O4: K6) strain.

4. Use according to anyone of claims from 1 to 3 wherein said active portion is the active site having the amino acid sequence from 720 to 1007 amino acid of CNF1 reference sequence CAA50007 (Gene Bank).

5. Use of a nucleic acid construct comprising an oligonucleotidic sequence encoding for a CNF1 protein toxin as defined according to anyone of the claims from 1 to 4 for the preparation of analgesic medicament.

6. Use according to anyone of the preceding claims for the treatment of acute or chronic, inflammatory, neoplastic pathologies, or post-operative conditions.

7. Use according to claim 6, wherein said inflammatory pathology is neuropathy.

8. Use according to claim 6, wherein said inflammatory pathology is neurodegenerative pathology.

9. Use according to anyone of the preceding claim, wherein said CNF1 bacterial protein toxin as defined in claims from 1 to 4, is used in association with at least an analgesic.

10. Use according to claim 9, wherein said analgesic is opioid.

11. Use according to claim 10, wherein said opioid is selected from the group consisting of morphine, codeine, dihydroxycodeine, diacetylmorphine, hydrocodone, hydromorphone, levorphanol, oxymorphone, alfentanyl, buprenorphine, butorphanol, fentanyl, sufentanyl, meperidine, methadone, nabulphine, propoxyphene, pentazocine; or pharmacologically acceptable salts thereof.

12. Use according to claim 11, wherein said opioid is morphine.

13. Pharmacological combination of active principles comprising CNF1 bacterial protein toxin as defined in claims from 1 to 4 or a nucleic acid construct as defined in claim 5 and at least an analgesic.

14. Pharmacological combination according to claim 13 wherein said analgesic is an opioid selected from the group consisting of morphine, codeine, dihydroxycodeine, diacetylmorphine, hydrocodone, hydromorphone, levorphanol, oxymorphone, alfentanyl, buprenorphine, butorphanol, fentanyl, sufentanyl, meperidine, methadone, nabulphine, propoxyphene, pentazocine; or pharmacologically acceptable salts thereof.

15. Pharmacological combination according to claim 14, wherein said opioid is morphine.

16. Pharmaceutical composition comprising the pharmaceutical combination as defined in anyone of claims from 13 to 15 together with one or more pharmaceutically acceptable adjuvants and/or excipients.

17. Pharmaceutical composition according to claim 16, wherein the toxin amount is variable in the range from 10 to 100 ng/ml/kg of patient body weight.

18. Use of the pharmacological combination according to anyone of claims from 13 to 15 or the pharmaceutical composition from 16 to 17 for the treatment of acute or chronic, inflammatory neoplastic pathologies, or post-operative conditions.

19. Use according to claim 18, wherein said inflammatory pathology is neuropathy.

20. Use according to claim 18 wherein said inflammatory chronic pathology is neurodegenerative pathology.

21. Use of antibodies specific for CNF1 bacterial protein toxin as defined in claims from 1 to 4 for the preparation of an antidote for the reduction of the analgesic effect of said dermonecrotizing protein factor.

22. Use according to claim 21, wherein said antibodies are monoclonal or polyclonal or antibody fragments

## Patentansprüche

1. Verwendung eines bakteriellen CNF1-Proteintoxins von einem nativen oder rekombinanten *Escherichia coli*-Stamm oder eines aktiven Teils oder einer aktiven Variante davon zur Herstellung eines analgetischen Medikaments.

2. Verwendung nach Anspruch 1, wobei der *Escherichia coli*-Stamm aus der Gruppe aus intestinalen und extra-intestinalen toxinbildenden Pathogenen ausgewählt ist.

3. Verwendung nach Anspruch 2, wobei der Stamm ein Uropathogen, vorzugsweise der Uropathogen-Stamm J96(04:K6), ist.

4. Verwendung nach irgendeinem der Ansprüche 1 bis 3, wobei der aktive Teil die aktive Stelle mit der Aminosäuresequenz von Aminosäure 720 bis 1007 der CNF1-Referenzsequenz CAA50007 (Gene Bank) ist.

5. Verwendung eines Nukleinsäurekonstrukts, umfassend eine Oligonukleotidsequenz, die für ein CNF1-Proteintoxin wie in irgendeinem der Ansprüche 1 bis 4 definiert codiert, zur Herstellung eines analgetischen Medikaments.

6. Verwendung nach irgendeinem der vorhergehenden Ansprüche zur Behandlung von akuten oder chronischen entzündlichen Erkrankungen, neoplastischen Erkrankungen oder post-operativen Zuständen.

7. Verwendung nach Anspruch 6, wobei die entzündliche Erkrankung eine Neuropathie ist.

8. Verwendung nach Anspruch 6, wobei die entzündliche Erkrankung eine neurodegenerative Erkrankung ist.

9. Verwendung nach irgendeinem der vorhergehenden Ansprüche, wobei das bakterielle CNF1-Proteintoxin wie in den Ansprüchen 1 bis 4 definiert in Verbindung mit mindestens einem Analgetikum verwendet wird.

10. Verwendung nach Anspruch 9, wobei das Analgetikum ein Opioid ist.

11. Verwendung nach Anspruch 10, wobei das Opioid aus der Gruppe, die aus Morphin, Codein, Dihydroxycodein, Diacetylmorphin, Hydrocodon, Hydromorphon, Levorphanol, Oxymorphon, Alfentanyl, Buprenorphin, Butorphanol, Fentanyl, Sufentanyl, Meperidin, Methadon, Nabulphin, Propoxyphen, Pentazocin oder pharmazeutisch annehmbaren Salzen davon besteht, ausgewählt ist.

12. Verwendung nach Anspruch 11, wobei das Opioid Morphin ist.

13. Pharmakologische Kombination von Wirkstoffen, umfassend ein bakterielles CNF1-Proteintoxin wie in den Ansprüchen 1 bis 4 definiert oder ein Nukleinsäurekonstrukt wie in Anspruch 5 definiert und mindestens ein Analgetikum.

14. Pharmakologische Kombination nach Anspruch 13, wobei das Analgetikum ein Opioid ist, welches aus der Gruppe, die aus Morphin, Codein, Dihydroxycodein, Diacetylmorphin, Hydrocodon, Hydromorphon, Levorphanol, Oxymorphon, Alfentanyl, Buprenorphin, Butorphanol, Fentanyl, Sufentanyl, Meperidin, Methadon, Nabulphin, Propoxyphen, Pentazocin oder pharmazeutisch annehmbaren Salzen davon besteht, ausgewählt ist.

15. Pharmakologische Kombination nach Anspruch 14, wobei das Opioid Morphin ist.

16. Pharmazeutische Zusammensetzung, umfassend die pharmazeutische Kombination wie in irgendeinem der Ansprüche 13 bis 15 definiert zusammen mit einem oder mehreren pharmazeutisch annehmbaren Adjuvanzien und/oder Exzipienten.

17. Pharmazeutische Zusammensetzung nach Anspruch 16, wobei die Toxinmenge im Bereich von 10 bis 100 ng/ml/kg Körpergewicht des Patienten variabel ist.

18. Verwendung der pharmakologischen Kombination nach irgendeinem der Ansprüche 13 bis 15 oder der pharmazeutischen Zusammensetzung nach den Ansprüchen 16 bis 17 zur Behandlung von akuten oder chronischen entzündlichen Erkrankungen, neoplastischen Erkrankungen oder post-operativen Zuständen.

19. Verwendung nach Anspruch 18, wobei die entzündliche Erkrankung eine Neuropathie ist.

20. Verwendung nach Anspruch 18, wobei die entzündliche chronische Erkrankung eine neurodegenerative Erkrankung ist.

21. Verwendung von Antikörpern, die für ein bakterielles CNF1-Proteintoxin wie in den Ansprüchen 1 bis 4 definiert spezifisch sind, zur Herstellung eines Gegenmittels für die Verringerung der analgetischen Wirkung des dermonekrotischen Proteinfaktors.

22. Verwendung nach Anspruch 21, wobei die Antikörper monoklonale oder polyklonale Antikörper oder Antikörperfragmente sind.

## Revendications

1. Utilisation d'une toxine protéique bactérienne CNF1 provenant d'une souche d'*Escherichia coli* native ou recombinante, d'une variante ou d'une portion active de celle-ci, pour la préparation d'un médicament analgésique.

2. Utilisation selon la revendication 1, où ladite souche d'*Escherichia coli* est choisie dans le groupe des pathogènes toxicogènes intestinaux et extra-intestinaux.

3. Utilisation selon la revendication 2, où ladite souche est un agent pathogène des voies urinaires, de préférence la souche d'agent pathogène des voies urinaires J96 (O4: K6).

4. Utilisation selon l'une quelconque des revendications 1 à 3, où ladite portion active est le site actif ayant la séquence d'acides aminés allant de l'acide aminé 720 à l'acide aminé 1007 de la séquence de référence CAA50007 de CNF1 (Gene Bank).

5. Utilisation d'une construction d'acide nucléique comprenant une séquence oligonucléotidique codant pour une toxine protéique CNF1 telle que définie selon l'une quelconque des revendications 1 à 4 pour la préparation d'un médicament analgésique.

6. Utilisation selon l'une quelconque des revendications précédentes pour le traitement de pathologies néoplasiques, inflammatoires, aiguës ou chroniques, ou d'affections postopératoires.

7. Utilisation selon la revendication 6, où ladite pathologie inflammatoire est une neuropathie.

8. Utilisation selon la revendication 6, où ladite pathologie inflammatoire est une pathologie neurodégénérative.

9. Utilisation selon l'une quelconque des revendications précédentes, où ladite toxine protéique bactérienne CNF1 telle que définie dans les revendications 1 à 4 est utilisée en association avec au moins un analgésique.

10. Utilisation selon la revendication 9, où ledit analgésique est un opioïde.

11. Utilisation selon la revendication 10, où ledit opioïde est choisi dans le groupe constitué par la morphine, la codéine, la dihydroxycodéine, la diacétylmorphine, l'hydrocodone, l'hydromorphone, le lévorphanol, l'oxymorphone, l'alfentanyl, la buprénorphine, le butorphanol, le fentanyl, le sufentanyl, la mépéridine, la méthadone, la nabulphine, le propoxyphène, la pentazocine, ou leurs sels acceptables sur le plan pharmacologique.

12. Utilisation selon la revendication 11, où ledit opioïde est la morphine.

13. Combinaison pharmacologique de principes actifs, comprenant une toxine protéique bactérienne CNF1 telle que définie dans les revendications 1 à 4 ou une construction d'acide nucléique telle que définie dans la revendication 5 et au moins un analgésique.

14. Combinaison pharmacologique selon la revendication 13, dans laquelle ledit analgésique est un opioïde choisi dans le groupe constitué par la morphine, la codéine, la dihydroxycodéine, la diacétylmorphine, l'hydrocodone, l'hydromorphone, le lévorphanol, l'oxymorphone, l'alfentanyl, la buprénorphine, le butorphanol, le fentanyl, le sufentanyl, la mépéridine, la méthadone, la nabulphine, le propoxyphène, la pentazocine, ou leurs sels acceptables sur le plan pharmacologique.

15. Combinaison pharmacologique selon la revendication 14, dans laquelle ledit opioïde est la morphine.

16. Composition pharmaceutique comprenant la combinaison pharmacologique telle que définie dans l'une quelconque des revendications 13 à 15, conjointement avec un ou plusieurs adjuvants et/ou excipients pharmaceutiquement acceptables.

17. Composition pharmaceutique selon la revendication 16, dans laquelle la quantité de toxine peut varier dans l'intervalle de 10 à 100 ng/ml/kg de poids corporel du patient.

18. Utilisation de la combinaison pharmacologique selon l'une quelconque des revendications 13 à 15 ou de la composition pharmaceutique des revendications 16 à 17 pour le traitement de pathologies néoplasiques inflammatoires, aiguës ou chroniques, ou d'affections postopératoires.

19. Utilisation selon la revendication 18, où ladite pathologie inflammatoire est une neuropathie.

20. Utilisation selon la revendication 18, où ladite pathologie inflammatoire chronique est une pathologie neurodégénérative.

21. Utilisation d'anticorps spécifiques de la toxine protéique bactérienne CNF1 telle que définie dans les revendications 1 à 4 pour la préparation d'un antidote destiné à réduire l'effet analgésique dudit facteur protéique dermonécrosant.

22. Utilisation selon la revendication 21, où lesdits anticorps sont des anticorps monoclonaux ou polyclonaux ou des fragments d'anticorps.
